# EUROPEAN PATENT SPECIFICATION

(11) **EP 2 906 540 B1**
(45) Date of publication and mention of the grant of the patent: **04.07.2018**
(21) Application number: 13782935.4
(22) Date of filing: 14.10.2013
(51) Int. Cl.: C07D 215/56

(54) **AN IMPROVED PRODUCTION METHOD AND NEW INTERMEDIATES OF SYNTHESIS OF ELVITEGRAVIR**
VERBESSERTES HERSTELLUNGSVERFAHREN UND NEUE ZWISCHENPRODUKTE DER SYNTHESE VON ELVITEGRAVIR
PROCÉDÉ DE PRODUCTION AMÉLIORÉ ET NOUVEAUX INTERMÉDIAIRES DE SYNTHÈSE D'ELVITÉGRAVIR

(30) Priority: 12.10.2012 CZ 20120698
(43) Date of publication of application: 19.08.2015
(73) Proprietor: Zentiva K.S., 102 37 Praha 10 (CZ)
(72) Inventor: RADL, Stanislav, 250 84 Praha-Kvetnice (CZ)
(74) Representative: Jirotkova, Ivana
(86) International application number: PCT/CZ2013/000129
(87) International publication number: WO 2014/056465

(56) References cited:
- US-A1- 2009 318 702

## Description

### Technical Field

The present invention relates to an improved method of producing elvitegravir (I), 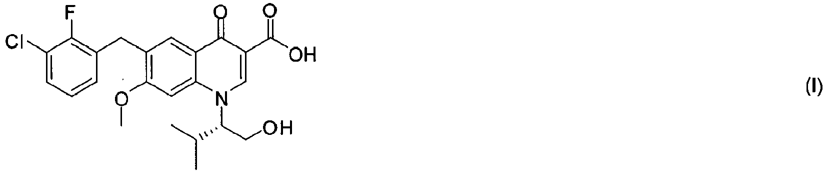 which is currently in Phase III clinical trials for the treatment of HIV infection. The drug was discovered by the company Japan Tobacco and was licensed by the company Gilead Sciences, which is carrying out the clinical development.

### Background Art

In the basic patent of the company Japan Tobacco, Inc. (US 7,176,220; EP 1,564,210; WO 2004/046115) two analogous synthetic procedures are described, starting from 2,4-difluorobenzoic acid **1.** According to the first procedure described by Scheme 1 this starting compound is first converted to benzoyl acrylate **5** in several stages. The latter is substituted with (S)-(+)-valinol **6** to the enamine **7,** which is subsequently cyclized to the quinolone **8,** whose free hydroxyl group is protected by a reaction with methyl chloroformate to the carbonate **9a.** In the next stage the carbonate reacts with 3-chloro-2-fluorobenzylzinc bromide **11** (prepared from 3-chloro-2-fluorobenzyl bromide **10** under the conditions of the Negishi coupling) in the presence of an organopalladium catalyst to the protected benzyl quinolone derivative **12a.** In the next stage the benzyl quinolone undergoes alkaline deprotection to the intermediate **13,** which is, in the last stage, subjected to a reaction with sodium methoxide to the end product Elvitegravir (Scheme 1).

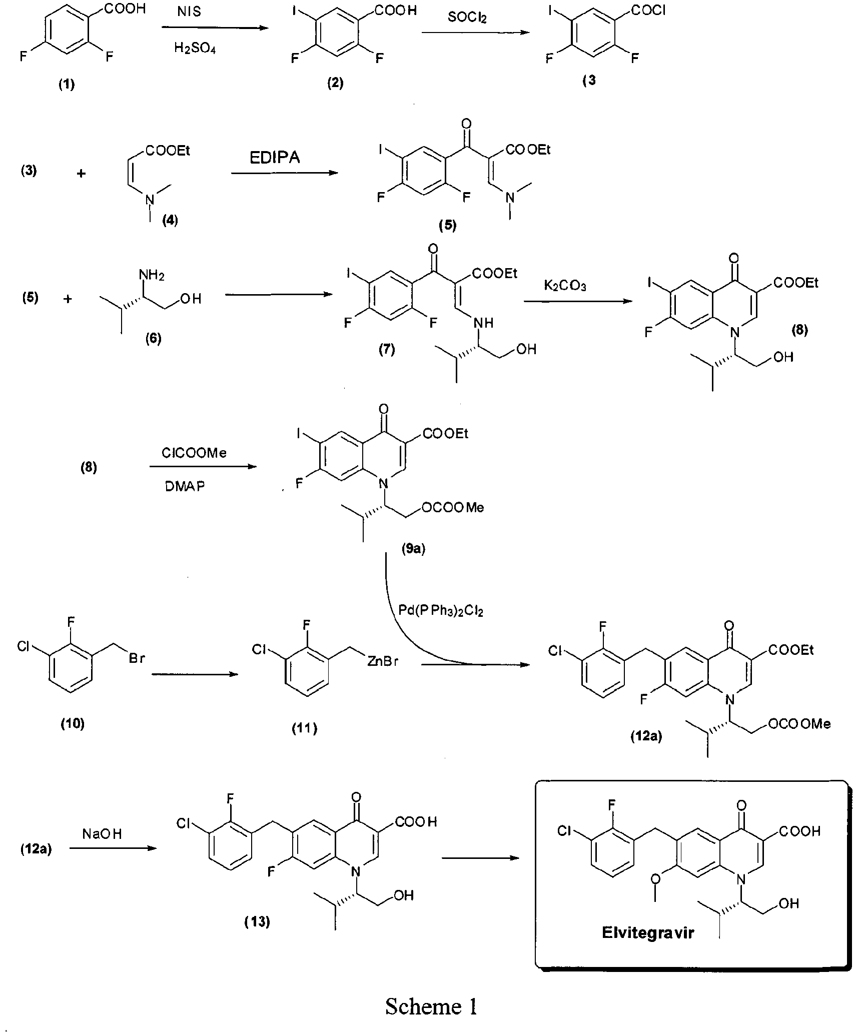

The other procedure described in the basic patent is the same as that described in Scheme 1 up to the intermediate **8,** whose hydroxyl group is protected with *tert*-butyldimethylsilyl chloride. The thus obtained protected silyl ether **9b** is further subjected to the Negishi coupling with 3-chloro-2-fluorobenzylzinc bromide **11** in the presence of bis(dibenzylidene acetone)palladium(0) and tris(2-furyl)phosphine to the protected intermediate **12b.** The remaining procedure is the same as in the previous synthetic route and includes alkaline deprotection to the hydroxy acid **13,** followed by a reaction with sodium methoxide (Scheme 2).

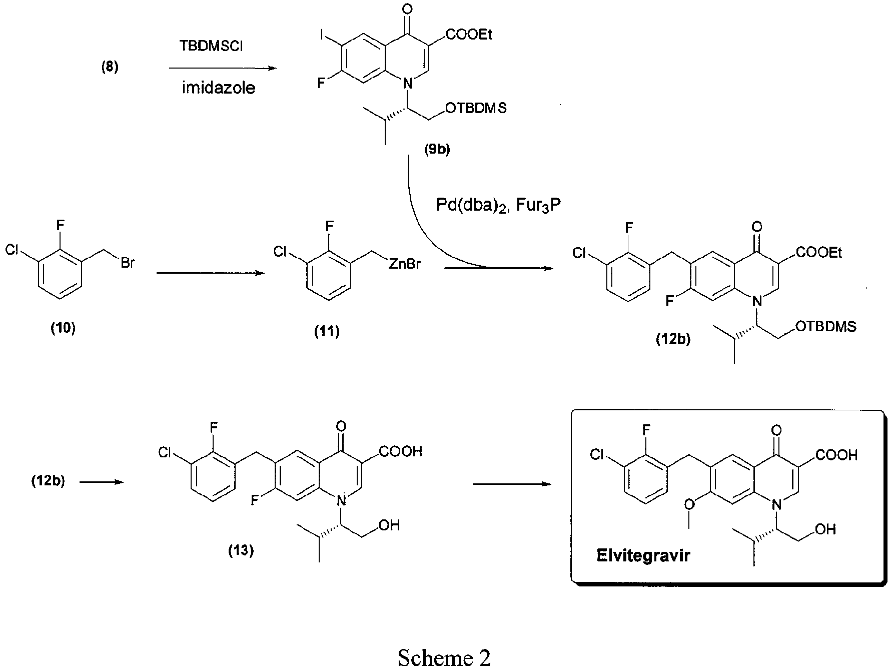

The patent application of the company Matrix Laboratories WO 2011/004389 describes synthesis that is analogous to the procedure of the basic patent, using protection of the hydroxyl by means of a tetrahydro pyranyl group (Scheme 3).

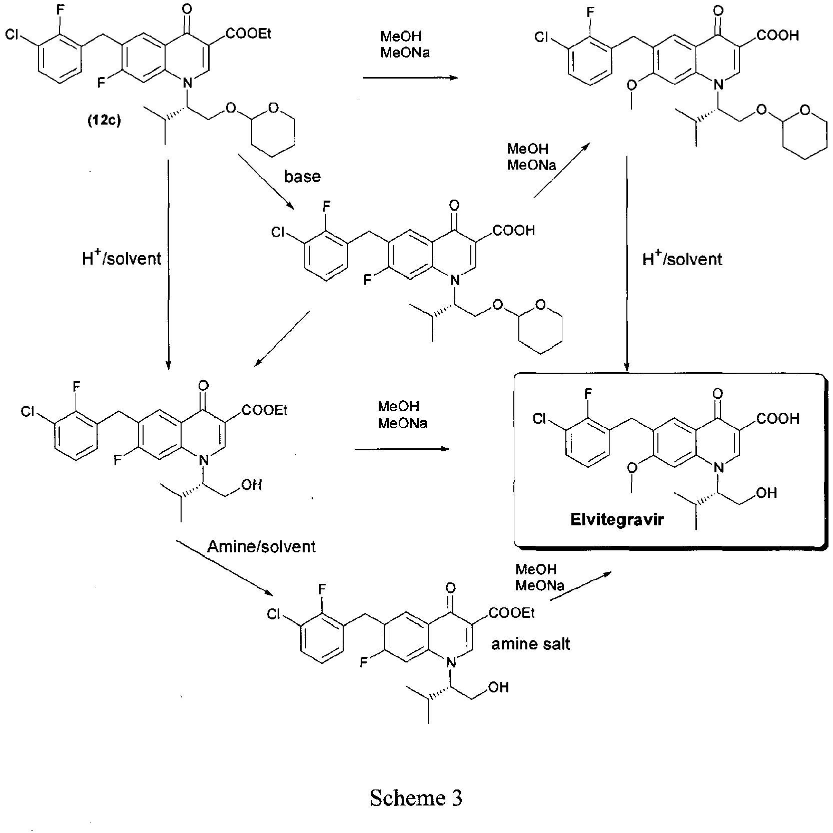

The more recent patent of the company Gilead Sciences US 7,825,252 (Scheme 3) starts from 2,4-dimethoxybenzoic acid **14.** This is converted, through a sequence of reactions, to the methyl ester **16,** whose reaction with 3-chloro-2-fluorobenzylzinc bromide **11** provides the ester **17,** which is converted to the β-ketoester **20** in the subsequent steps. Its reaction with DMF-dimethylacetal provides the benzoacrylate **21,** which further reacts with (S)-(+)-valinol **6** to provide the enamine **22.**

In the next step cyclization of the enamine **22** to the quinolinone **24** is carried out. Generally, similar cyclizations leading to the quinolone skeleton are usually carried out with compounds that have fluorine, chlorine or a nitro group as leaving groups in position 2 (for general information, see Heterocycles 1992, 34, 2143-2177; Adv. Het. Chem. 2002, 83, 189-257). In spite of this, a few cases are described where as the leaving group a methoxy group is applied. The oldest reference to a similar reaction (Liebigs Ann. Chem. 1987, 29-37; Ger. Offen., 3501247) describes that heating with potash in DMF was used for cyclization. More recent patent applications of the company Procter & Gamble (WO 2007/110834; WO 2007/110835) describe cyclization of similar 2-methoxy derivatives by heating with *O*,*N*-bis-trimethylsilyl acetamide in toluene. Use of this method for synthesis of elvitegravir is then described in the applications WO 2009/089263 and US 2009/0318702. In the next stage its hydroxyl group is protected with a reaction with *tert*-butyldimethylsilyl chloride and the resulting compound **23** is subsequently circularized to the protected quinolone derivative **24.** The last stage consists of hydrolysis of the ethyl ester as well as removal of the protecting TBDMS group (Scheme 4).

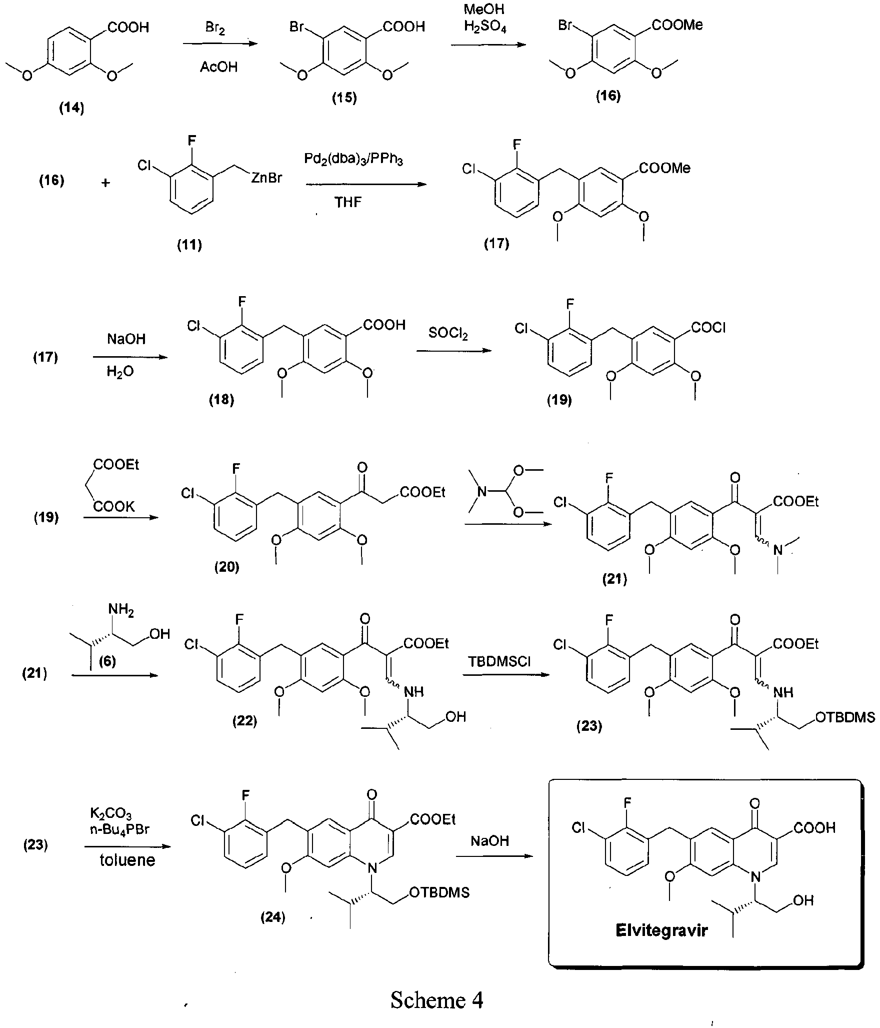

The procedure described in the process patent of the company Gilead Sciences US 7,825,252 uses a similar synthesis of the quinolone ring to the above mentioned procedures; however, with a different method of building up the key intermediates (Scheme 5). In the first step of this synthesis the bromoacid **15** is first converted to the magnesium salt and after subsequent addition of butyllithium the resulting salt reacts with the substituted benzaldehyde **25** to the hydroxy acid **26.** In the next step the hydroxy group is reduced with the use of triethylsilane to the acid **18.** The acid is then converted, through a reaction with carbonyldiimidazole, to the imidazole functional derivative **27,** which subsequently reacts with potassium ethylmalonate to the β-ketoester **20**. The remaining steps are the same as in the above mentioned procedures (Scheme 5).

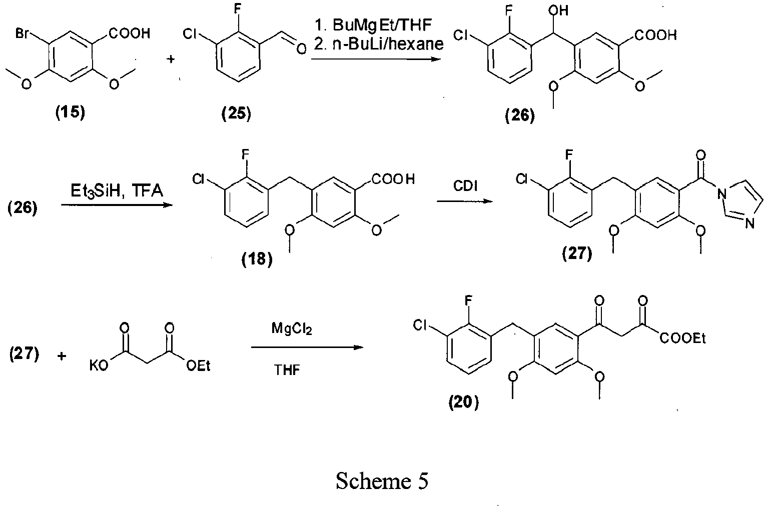

### Disclosure of Invention

This invention provides a method of producing elvitegravir of formula **I** 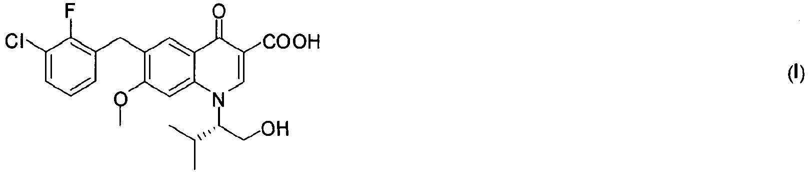 which starts from dimethoxy derivatives of the general formula **II,** 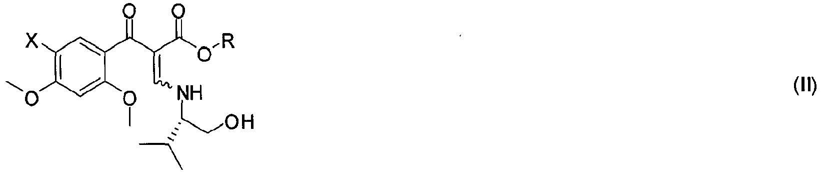 wherein X is Cl, Br, or I, and wherein R is an (un)branched C₁-C₄ alkyl, which provide, through an *in situ* reaction with a suitable silylation reagent in a solvent, after processing and isolation, the corresponding silylated intermediate **III**, 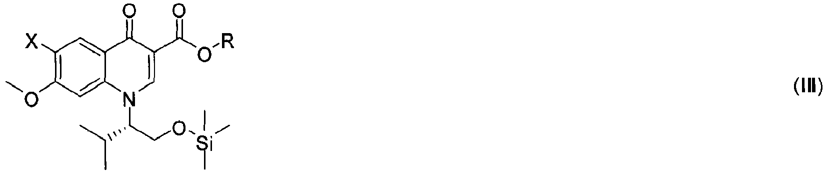 wherein X and R have the above mentioned meaning, which then, through a reaction with the organozinc reagent **IV,** 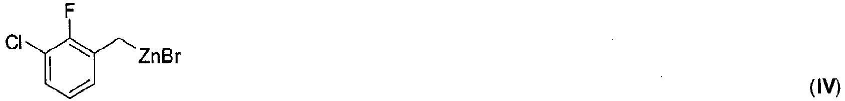 provides an intermediate of the general formula **V,** 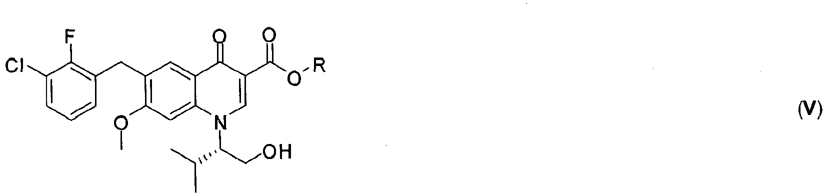 wherein R is an (un)branched C₁-C₄ alkyl, which can be optionally crystallized from a crystallization solvent and is further hydrolyzed to elvitegravir of formula **I,** which is optionally crystallized.

The key reaction of the entire sequence of elvitegravir synthesis is cyclization of the compounds II, wherein the methoxy group acts as the leaving group (Scheme 6).

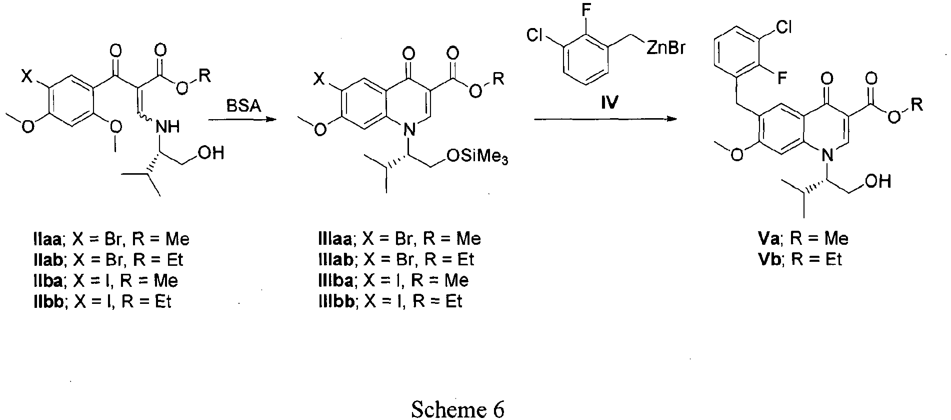

A reproduction of conditions described in literature for the compound **IIaa** *(*Liebigs Ann. Chem. 1987, 29-37; Ger. Offen., 3501247) with the use of heating with potash in DMF provided a mixture containing, *inter alia,* the elimination product **VI** and dimer **VII** (both the compounds identified with LC-MS).

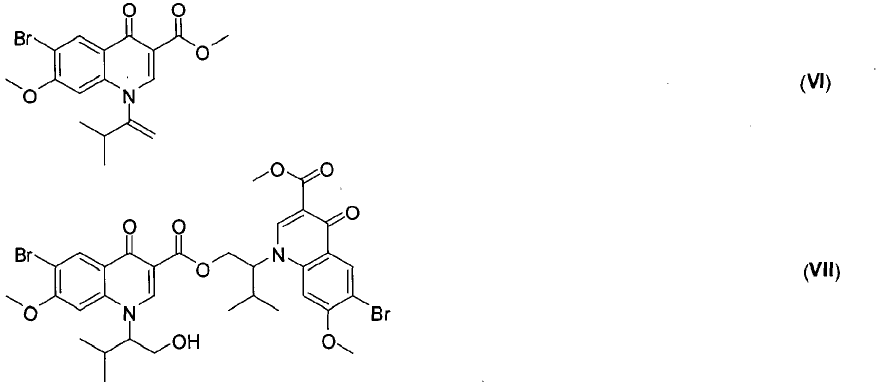

So it is evident that the hydroxyl group in the intermediates **II** has to be protected with a suitable protecting group before the cyclization. As follows from analysis of the prior art, a number of protecting groups are used, including the *tert*-butyldimethylsilyl group. As this analysis shows, the cheaper trimethylsilyl protecting group was considered to be insufficiently stable to remain in the product **III** during processing and to be able to be used as a protecting group in the next reaction step as well, i.e., in the case of synthesis of elvitegravir (**I**), the coupling with the organozinc reagent **IV.** The patent WO 2009/089263, e.g., describes cyclization of the compound **IIab** with the use of *O*,*N*-bis-trimethylsilylacetamide, where the authors after the reaction isolated the corresponding hydroxy derivative **VIII,** which was then converted, through a reaction with *tert*-butyldimethylsilyl chloride, to the protected intermediate **IX.** On the other hand, in the application US 2009/0318702 the free hydroxyl group in the intermediate **IIab** is first protected by the *tert*-butyldimethylsilyl group and the cyclization is executed after that.

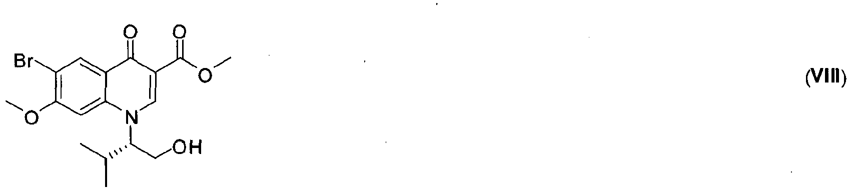

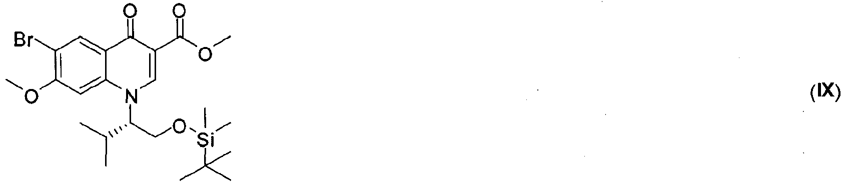

With regard to the above mentioned procedure of cyclization with *O*,*N-*bis-trimethylsilylacetamide and subsequent protection of the free hydroxyl group with *tert-*butyldimethylsilyl chloride we assumed that either the corresponding *O*-trimethylsilyled derivative could not be isolated due to insufficient stability or it could not be used for a reaction with an organozinc reagent. However, we have surprisingly found out that the silylated product **IIIaa** primarily produced by cyclization of the compound **IIaa** can be isolated by common careful processing. Analogously, we have found out that similar intermediates III can also be isolated, e.g., the intermediates IIIab, IIIba and IIIbb.

The silylated intermediate **III** is processed and isolated in such a way that the reaction mixture is cooled to a temperature in the range of 0 to 5°C, a sufficient amount of water is added dropwise so that precipitate can be formed, which is filtered off and dried. The separated precipitate can be preferably washed with water before drying and then it is dried in vacuum at the room temperature.

The resulting silylated products **III** are sufficiently stable to be isolated and subsequently used for a reaction with an organozinc reagent. In such a reaction, e.g., the intermediate **IIIaa** or **IIIab** is converted to the cyclized derivative **Va** by reacting with (3-chloro-2-fluorobenzyl)zinc bromide **IV,** the reaction being catalyzed by a suitable Pd catalyst.
Using the same method the compound **Vb** was prepared from the intermediate **IIIba** or **IIIbb.** The compounds of formula **V** can be, without problems, crystallized from solvents such as toluene or C₁-C₃ (un)branched alcohols or a mixture of toluene with these alcohols, preferably a toluene-methanol mixture.
Their conversion to elvitegravir is possible with the use of known procedures. The same sequence of reactions was also performed with the other intermediates **II.**

This modification makes it possible to shorten the process by at least one reaction stage and in addition it avoids using the expensive *tert*-butyldimethylsilyl chloride. Moreover, the 3-chloro-2-fluorobenzyl group is only introduced in the molecule in the final reaction stages, which considerably reduces material costs with regard to the high price of the respective 3-chloro-2-fluorobenzylhalo derivatives.

As regards the accomplishment of the cyclization, we have found out that the presence of more than 2 equivalents of the silylation reagent is necessary to achieve virtually complete conversion (HPLC). Out of the range of the tested silylation reagents it is in particular *O*,*N-*bis-trimethylsilyltrifluoroacetamide and *N*-methyltrimethylsilylacetamide that have proven to be efficient besides *O,N*-bis-trimethylsilylacetamide. For cost reasons *O,N*-bis-trimethylsilylacetamide was selected as the most suitable compound for the optimization and the reaction was carried out in DMF. It was mainly the reaction of (*S*)-methyl 3-(1-hydroxy-3-methylbutan-2-ylamino)-2-(5-bromo-2,4-dimethoxy-benzoyl)-acrylate (**IIaa**) that was optimized. We have found out that first when using ca. 2.2 fold molar excess complete conversion is achieved and that first when using ca. 2.4 fold molar excess the reaction runs in a sufficiently clean manner. Monitoring of the reaction at the laboratory temperature (TLC, LC-MS) showed that after addition of the reagent (in an amount from 1 equivalent to 2.5 equivalents) the starting compound quickly disappeared and the respective O-TMS derivative was formed. However, when 2.5 equivalents were used, complete conversion to the desired product did not even occur after 48 hours at the laboratory temperature. What proved to be convenient for cleanness and speed of the reaction was the use of *O,N*-bis-trimethylsilylacetamide in an amount in the range of 2.2 - 5 equivalents, preferably in the range of 2.2- 3.0 equivalents and carrying out the reaction in such a way that after addition of the silylation agent the reaction mixture was first stirred for a shorter time (15 minutes up to 2 hours) at the laboratory temperature and then the cyclization itself was executed at an increased temperature in the range from 60°C to the boiling point of the mixture, preferably at temperatures from 80 °C to 120 °C. Carrying out the reaction at 100 °C turned out to be optimal from in terms of the reaction time, purity and yield of the product.

In the effort to achieve more cost savings we focused on the possibility to first carry out trimethylsilylation with a suitable silylation reagent at the laboratory temperature and to perform the cyclization itself of the primarily produced silyled intermediate **X** by heating with a suitable base after that.

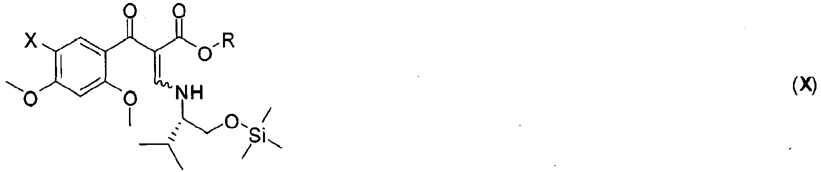

During the optimization of this process we executed the reaction in DMF with the use of 1 to 1.5 equivalents of *O,N*-bis-trimethylsilylacetamide at the laboratory temperature and then a suitable base was added and the cyclization itself was performed at an increased temperature. As the increased temperature, we again selected a temperature in the range from 60°C to the boiling point of the reaction mixture again while the temperature of 100°C appeared again to be optimal. Alkali metal carbonates or hydrogen carbonates (Na₂CO₃, NaHCO₃, K₂CO₃, LiCO₃), alkali metal (lithium, sodium or potassium) hexamethyldisilazides, or organic bases, e.g. diisopropylethylamine (DIPEA), 8-diazabicyclo[5.4.0.]undec-7-en (DBU), 1,4-diazabicyclo[2.2.2]octane (DABCO), 1,8-bis-(dimethylamino)naphthalene, 1-methylmorpholine, 1-ethylpiperidine, were tested as suitable bases. Especially when carbonates of alkaline metals and sodium hexamethyldisilazide were used, the speed of the cyclization was comparable to the use of a surplus of *O,N*-bis-trimethylsilylacetamide, however the free hydroxy derivative **VIII** was formed to a higher extent. So this option would rather be attractive for synthesis of the hydroxy derivative **VIII.** The use of strong organic bases provided similar results - but in this case the reaction was must slower and the reaction mixture contained other unidentified products according to HPLC.

The compounds **V** prepared in accordance with this invention are then hydrolyzed to elvitegravir of formula **I** 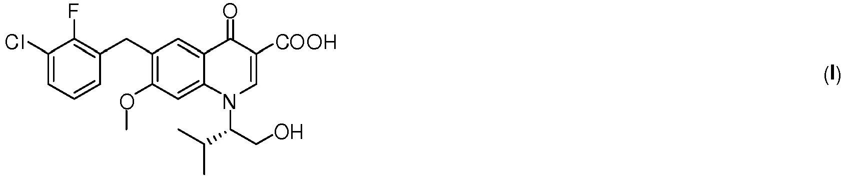 in an alkaline environment, e.g. they are hydrolyzed in a mixture of water : C₁-C₃ (un)substituted aliphatic alcohol in the presence of an alkali hydroxide, preferably in a water : methanol mixture in the presence of sodium hydroxide. The hydrolysis is carried out at a temperature in the range from 20°C to the boiling point of the mixed solvent, preferably at a temperature of 20 - 50 °C.

The obtained product (**I**) can then be crystallized from a mixture of a polar organic solvent and water, e.g. from a mixture of acetone and water.

The following compounds of formulae **IIIaa**, **IIIab**, **IIIba** and **IIIbb** are stable and are useful in the synthesis of elvitegravir of formula **I.**

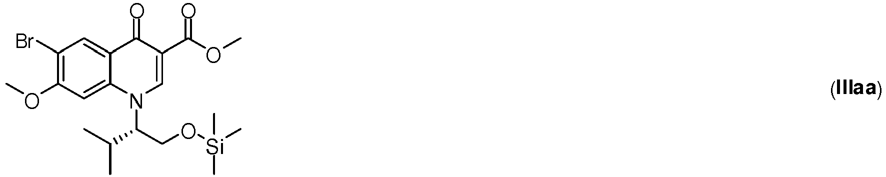

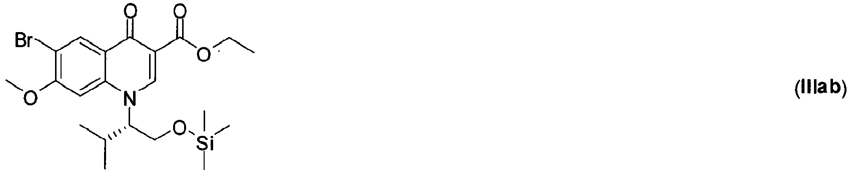

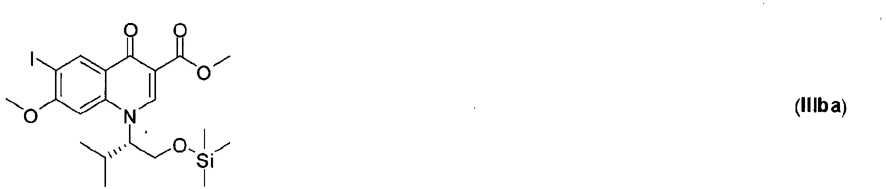

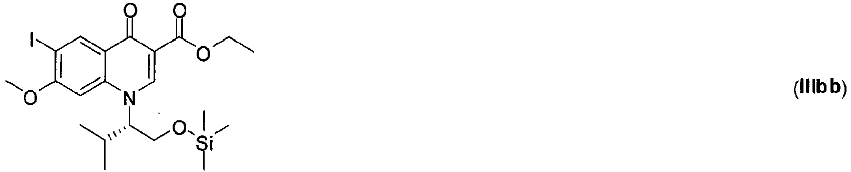

Using the method in accordance with this invention the (*R*)-enantiomer of elvitegravir of formula **XII** is also prepared 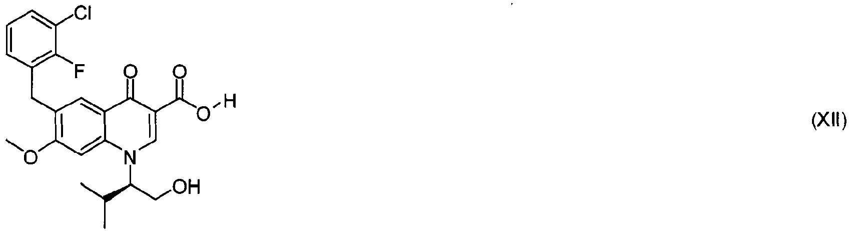 with the use of the compound of formula XI

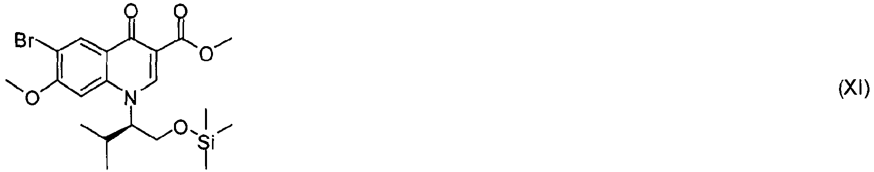

### Examples

The reactions were routinely monitored with the use of HPLC in an HP 1050 device equipped with a Phenomenex Luna 5µ C18(2), 250 x 4.6 mm column and a UV detector, 227 nm. Phase A: 1.2 g of NaH₂PO₄/1 1 of water (pH = 3.0); Phase B: methanol.

The invention is explained in a more detailed way with the use of the working examples below. These examples, which illustrate the improvement of the method in accordance with the invention, have an exclusively illustrative character and do not limit the scope of the invention in any respect.

### Example 1

### (S)-Methyl 6-bromo-7-methoxy-1-(3-methyl-1-(trimethylsilyloxy)butan-2-yl)-4-oxo-1,4-dihydroquinoline-3-carboxylate (IIIaa)

*O,N*-bis-Trimethylsilylacetamide (**BSA;** 20 g, 98 mmol) was added to a solution of (*S*)-methyl 3-(1-hydroxy-3-methylbutan-2-ylamino)-2-(5-bromo-2,4-dimethoxy-benzoyl)-acrylate (**IIaa**; 17.2 g, 40 mmol) in DMF (150 ml) and the mixture was first stirred at the laboratory temperature for 1 hour and then at 100°C under nitrogen for 1 hour. The mixture was cooled to the laboratory temperature and then, the mixture being cooled by a water-ice mixture, water (200 ml) was added dropwise. The separated precipitate was aspirated, washed with cold water and dried in vacuum at 20-22 °C. 18.5 g (98 %) of the compound **IIIa** with the melt. point of 193-196 °C and HPLC purity of 94.5 %, was obtained, which was used without further purification for the subsequent reaction described in Example 6. ¹H NMR (250 MHz, CDCl₃) δ (ppm): 8.72 (s, 1H, -CH=), 8.67 (s, 1H, H-5), 6.88 (s, 1H, H-8), 4.05-4,17 (m, 1H, CHN), 3.95-4.01 (m, 5H, MeO, CH₂OH), 3.93 (s, 3H, COOMe), 2.39-2.46 (m, 1H, CHMe₂), 1.18 (d, *J* = 6.4 Hz, 3H, CHMe₂), 0.84 (d, *J* = 6.4 Hz, 3H, CHMe₂), 0.01 (s, 9H, SiMe₃).

### Example 2

### (S)-Methyl 1-(1-hydroxy-3-methylbutan-2-yl)-6-bromo-7-methoxy-4-oxo-1,4-dihydroquinoline-3- carboxylate (VIII)

*O,N*-bis-Trimethylsilylacetamide (BSA; 2 g, 9.8 mmol) was added to a solution of (*S*)-methyl 3-(1-hydroxy-3-methylbutan-2-ylamino)-2-(5-bromo-2,4-dimethoxy-benzoyl)acrylate (**IIaa**; 1.72 g, 4 mmol) in DMF (15 ml) and the mixture was stirred at 100 °C under nitrogen for 1 hour. The mixture was cooled, a mixture of water (20 ml) and 5% HCl (1 ml) was added and the mixture was stirred at the laboratory temperature for 1 hour. White bulky precipitate was aspirated, washed with water and dried. The reaction provided 1.4 g (88 %) of the compound **VIII** with the melt. point of 141-145 °C and HPLC purity of 99.4 %. ¹H NMR (250 MHz, CDCl₃) δ (ppm): 8.56 (s, 1H, -CH=), 7.77 (s, 1H, H-5), 6.86 (s, 1H, H-8), 5.65 (t, *J* = 4.2 Hz, 1H, OH), 4.64-4.72 (m, 1H, CHN), 4.23-4.32 (m, 2H, CH₂OH), 4.14 (s, 3H, MeO), 3.86 (s, 3H, COOMe), 2.43-2,52 (m, 1H, CHMe₂), 1.24 (d, *J* = 6.4 Hz, 3H, CHMe₂), 0.72 (d, *J* = 6.4 Hz, 3H, CHMe₂).

### Example 3

### (S)-Methyl 1-(1-hydroxy-3-methylbutan-2-yl)-6-bromo-7-methoxy-4-oxo-1,4-dihydroquinoline-3- carboxylate (VIII)

Using the procedure described in Example 2 with the use of 4 equivalents of *N-*methyltrimethylsilyl-acetamide, 86 % of the compound **VIII** with the HPLC purity of 97.6 % was obtained.

### Example 4

### (S)-Methyl 1-(1-hydroxy-3-methylbutan-2-yl)-6-bromo-7-methoxy-4-oxo-1,4-dihydroquinoline-3- carboxylate (VIII)

*O,N*-bis-Trimethylsilyltrifluoroacetamide (0.55 g, 2.1 mmol) was added to a solution of (S)-methyl 3-(1-hydroxy-3-methylbutan-2-ylamino)-2-(5-bromo-2,4-dimethoxy-benzoyl)acrylate (**IIaa**; 0.43 g, 1 mmol) in DMF (5 ml) and the mixture was stirred at 100 °C under nitrogen for 1 hour. Then, the mixture was evaporated until dry in a vacuum evaporator, the oily substance was mixed with methanol (10 ml) and after addition of 5 % HCl (0.5 ml) the mixture was stirred at the laboratory temperature overnight. After that, water (10 ml) was added and the white bulky precipitate was aspirated, washed with water and dried. 0.27 g (68 %) of the compound **VIII** with the melt. point of 139-142 °C and HPLC purity of 98.7 % was obtained.

### Example 5

### (S)-Ethyl 6-bromo-7-methoxy-1-(3-methyl-1-(trimethylsilyloxy)butan-2-yl)-4-oxo-1,4-dihydroquinoline-3-carboxylate (IIIab)

Using the procedure described in Example 1 the compound **IIIab** with the melt. point of 154-174°C (decomp.) and HPLC purity of 96.6 % was obtained in the 92% yield, which was used without further purification for a subsequent reaction described in Example 10. ¹H NMR (250 MHz, CDCl₃) δ (ppm): 8.69 (s, 1H, -CH=), 8.39 (s, 1H, H-5), 7.41 (s, 1H, H-8), 4.27 (q, *J* = 7.1 Hz, 2H, OCH₂CH₃), 4.08 (s, 3H, MeO), 3.92-4.08 (m, 3H, CHN, CH₂OH), 2.37-2.48 (m, 1H, CHMe₂), 1.31 (t, *J* = 7.1 Hz, 3H, OCH₂CH₃), 1.17 (d, *J* = 6.4 Hz, 3H, CHMe₂), 0.79 (d, *J* = 6.4 Hz, 3H, CHMe₂), 0.01 (s, 9H, SiMe₃).

### Example 6

### (S)-Methyl 6-iodo-7-methoxy-1-(3-methyl-1-(trimethylsilyloxy)butan-2-yl)-4-oxo-1,4-dihydroquinoline-3-carboxylate (IIIba)

Using the procedure described in Example 1 the compound **IIIba** with the melt. point of 192-194°C and HPLC purity of 98.4 % was obtained in the 87% yield, which was used without further purification for a subsequent reaction described in Example 9. ¹H NMR (250 MHz, CDCl₃) δ (ppm): 8.94 (s, 1H, -CH=), 8.67 (s, 1H, H-5), 6.79 (s, 1H, H-8), 4.07-4.16 (m, 1H, CHN), 3.95-4.03 (m, 5H, MeO, CH₂OH), 3.92 (s, 3H, COOMe), 2.40-2.44 (m, 1H, CHMe₂), 1.18 (d, *J* = 6.4 Hz, 3H, CHMe₂), 0.84 (d, *J* = 6.4 Hz, 3H, CHMe₂), 0.08 (s, 9H, SiMe₃).

### Example 7

### (S)-Ethyl 6-iodo-7-methoxy-1-(3-methyl-1-(trimethylsilyloxy)butan-2-yl)-4-oxo-1,4-dihydroquinoline-3 -carboxylate (IIIbb)

Using the procedure described in Example 1 the compound **IIIbb** with the melt. point of 167-171°C and HPLC purity of 98.2 % was obtained in the 84% yield, which was used without further purification for a subsequent reaction described in Example 11. ¹H NMR (250 MHz, CDCl₃) δ (ppm): 8.65 (s, 1H, -CH=), 8.58 (s, 1H, H-5), 7.26 (s, 1H, H-8), 4.24 (q, *J=* 7.1 Hz, 2H, OCH₂CH₃), 4.02 (s, 3H, MeO), 3.89-3.96 (m, 3H, CHN, CH₂OH), 2.32-2.46 (m, 1H, CHMe₂), 1.27 (t, *J* = 7.1 Hz, 3H, OCH₂CH₃), 1.16 (d, *J* = 6.4 Hz, 3H, CHMe₂), 0.75 (d, *J* = 6.4 Hz, 3H, CHMe₂), 0.01 (s, 9H, SiMe₃).

### Example 8

### (S)-Methyl 6-(3-chloro-2-fluorobenzyl)-1-(1-hydroxy-3-methylbutan-2-yl)-7-methoxy-4-oxo-1,4-dihydroquinoline-3-carboxylate (Va)

*Preparation of (3*-*chloro-2-fluorobenzyl)zinc bromide*: A solution of 1,2-dibromoethane (0.1 g) and trimethylsilyl chloride (0.5 ml) in dry THF (1.5 ml) was prepared. 0.2 ml was withdrawn from this solution and this fraction was added to a suspension of granulated Zn (20-30 mesh; 0.62 g) in dry THF (2.5 ml) stirred under Ar at 60 °C. After 30 minutes a solution of (3-chloro-2-fluorobenzyl)bromide (2.23 g, 10 mmol) was added with a linear dispenser during 2 hours and the mixture was stirred at the above mentioned temperature for another 2 hours, while virtually all the metal dissolved.

*Coupling itself:* PdCl₂(Ph₃P)₂ (0,2 g) was added to a solution of (*S*)-methyl 6-bromo-7-methoxy-1-(3-methyl-1-(trimethylsilyl-oxy)butan-2-yl)-4-oxo-1,4-dihydroquinoline-3-carboxylate (**IIIaa**; 3.33 g, 7 mmol) in dry THF (35 ml), the mixture was heated under Ar to 60 °C and the solution of (3-chloro-2-fluorobenzyl)zinc bromide prepared using the above mentioned procedure was added using a linear dispenser at this temperature during one hour and the mixture was stirred at this temperature for another 1 h. After cooling the mixture was poured into a separating funnel containing a saturated solution of NH₄Cl (50 ml) and ethyl acetate (50 ml). After thorough shaking the aqueous layer was let out and the organic layer was gradually washed with a saturated solution of NH₄Cl (20 ml), saturated solution of NaHCO₃ (20 ml) and brine (2 + 20 ml). The resulting dark solution was stirred with Carborafin and after its aspiration through kieselguhr a yellowish solution was obtained, after the evaporation of which the obtained evaporation product solidified (4.5 g). This evaporation product was dissolved in methanol (50 ml) and after addition of 5% HCl (0.5 ml) the mixture was stirred at the laboratory temperature for 24 h. During stirring white precipitate gradually separated from the solution. Then, the mixture was cooled in a refrigerator, the insoluble fraction was aspirated. 2.8 g (61 %) of a substance was obtained containing 94 % of the compound **IIa** according to HPLC. Crystallization from toluene containing 5 % of MeOH provided 2.1 g of the substance with the melt. point of 192-194 °C and HPLC content of 98.3 %. ¹H NMR (250 MHz, CDCl₃) δ (ppm): 8.65 (s, 1H, -CH=), 7.90 (s, 1H, H-5), 7.29 (t, 1H, Ar-H), 7.22 (s, 1H, H-8), 7.15-7.23 (m, 2H, Ar-H), 5.13 (t, *J* = 4.6 Hz, 1H, OH), 4.80-4.94 (m, 1H, CHN), 4.05 (s, 2H, CH₂), 3.92-3.98 (m, 5H, MeO, CH₂OH), 3.73 (s, 3H, COOMe), 2.29-2.40 (m, 1H, CHMe₂), 1.12 (d, *J* = 7.1 Hz, 3H, CHMe₂), 0.74 (d, *J* = 7,1 Hz, 3H, CHMe₂).

### Example 9

### (S)-Methyl 6-(3-chloro-2-fluorobenzyl)-1-(1-hydroxy-3-methylbutan-2-yl)-7-methoxy-4-oxo-1,4-dihydroquinoline -3- carboxylate (Va)

Using the procedure described in Example 8 with the use of the methyl ester of the iodine derivative **IIIab** instead of the methyl ester of the bromine derivative **IIIaa** the compound **Va** with the melt point of 193-195 °C and HPLC purity of 97.2 % was obtained in the 83% yield, which was used without further purification for a subsequent reaction described in Example 10.

### Example 10

### (S)-Ethyl 6-(3-chloro-2-fluorobenzyl)-1-(1-hydroxy-3-methylbutan-2-yl)-7-methoxy-4-oxo-1,4-dihydroquinoline-3-carboxylate (Vb)

Using the procedure described in Example 8 with the use of the ethyl ester of the bromine derivative **IIIbb** instead of the methyl ester of the bromine derivative **IIIaa** the compound **Vb** with the melt point of 178-183 °C and HPLC purity of 93.8 % was obtained in the 81% yield, which was used without further purification for a subsequent reaction described in Example 13. For the characterization a sample of the crude compound **Vb** was purified by crystallization in a mixture of toluene - ethanol 8 : 2. ¹H NMR (250 MHz, DMSO*d6*) δ (ppm): 8.62 (s, 1H, -CH=), 7.94 (s, 1H, H-5), 7.17-7.32 (m, 4H, Ar-H), 5.11 (t, *J* = 4.6 Hz, 1H, OH), 4.62-4.70 (m, 1H, CHN), 4.22 (q, *J* = 7.1 Hz, 2H, OCH₂CH₃), 4.04 (s, 2H, CH₂), 3.89-3.96 (m, 5H, MeO, CH₂OH), 2.36-2.45 (m, 1H, CHMe₂), 1.29 (t, *J* = 7.1 Hz, 3H, OCH₂CH₃), 1.15 (d, *J* = 7,1 Hz, 3H, CHMe₂), 0.76 (d, *J* = 7,1 Hz, 3H, CHMe₂).

### Example 11

### (S)-Ethyl 6-(3-chloro-2-fluorobenzyl)-1-(1-hydroxy-3-methylbutan-2-yl)-7-methoxy-4-oxo-1,4-dihydroquinoline-3-carboxylate (Vb)

Using the procedure described in Example 8 with the use of the ethyl ester of the iodine derivative **IIIbb** instead of the methyl ester of the bromine derivative **IIIaa** the compound **Vb** with the melt point of 178-183 °C and HPLC purity of 96.3 % was obtained in the 85% yield, which was used without further purification for a subsequent reaction described in Example 13.

### Example 12

### (S)-6-(3-Chloro-2-fluorobenzyl)-1-(1-hydroxy-3 -methylbutan-2-yl)-7-methoxy-4-oxo-1,4-dihydroquinoline-3- carboxylic acid - elvitegravir (I)

A solution of NaOH (5 g) in water (100 ml) was added to a stirred suspension of (*S*)-methyl 6-(3-chloro-2-fluorobenzyl)-1-(1-hydroxy-3-methylbutan-2-yl)-7-methoxy-4-oxo-1,4-dihydroquinoline-3-carboxylate (**Va;** 10 g, 21.6 mmol) in methanol (100 ml) and the mixture was stirred at the laboratory temperature for 15 hours. The mixture was diluted with water (250) and light turbidity was removed by filtration through a folded filter. The filtrate was then acidified with 10% HCl and the separated white precipitate was aspirated, washed with water and air-dried. 8.2 g (85 %) of the crude product was obtained with the melt. point of 195-202 °C and HPLC purity of 97.8 %. Crystallization of the sample from aqueous acetone provided HPLC purity of 99.8 while maintaining the melting point. ¹H NMR (250 MHz, DMSO*d6*) δ (ppm): 15.42 (s, 1H, COOH), 8.88 (s, 1H, -CH=), 8.05 (s, 1H, H-5), 7.44-7.49 (m, 2H, H-8, Ar-H), 7.15-7.28 (m, 2H, Ar-H), 5.18 (t, 1H, *J* = 4.6 Hz, OH), 4.80-4.92 (m, 1H, CHN), 4.11 (s, 2H, CH₂), 3.96-4.03 (m, 4H, MeO + CH₂OH), 3.77-3.82 (m, 1H, CH₂OH), 2.37-2.42 (m, 1H, CHMe₂), 1.16 (d, *J* = 6.4 Hz, 3H, CHMe₂), 0.74 (d, *J* = 6.4 Hz, 3H, CHMe₂).

### Example 13

### (S)- 6-(3-Chloro-2-fluorobenzyl)-1-(1-hydroxy-3-methylbutan-2-yl)-7-methoxy-4-oxo-1,4-dihydroquinoline-3- carboxylic acid - elvitegravir (I)

Using the procedure described in Example 12 with the use of the ethyl ester **Vb** instead of the methyl ester **Va** the compound **I** with the melt. point of 195-201°C (decomp.) and HPLC purity of 98.3 % was obtained in the 76% yield.

### Example 14

### (S)-Methyl 6-bromo-7-methoxy-1-(3-methyl-1-(trimethylsilyloxy)butan-2-yl)-4-oxo-1,4-dihydroquinoline-3-carboxylate (IIIaa)

*O,N*-bis-Trimethylsilylacetamide (**BSA**; 1.2 g, 6 mmol) was added to a solution of (*S*)-methyl 3-(1-hydroxy-3-methylbutan-2-ylamino)-2-(5-bromo-2,4-dimethoxy-benzoyl)-acrylate (**IIaa**; 2.0 g, 4.66 mmol) in DMF (20 ml) and the mixture was stirred at the laboratory temperature for 1 hour. Then, the mixture was divided and 2 ml each of this solution was pipetted into vials containing 0.1 g of a base (A - without base, B - BSA, C - NaHCO₃, D - KHCO₃, E - Na₂CO₃, F - K₂CO₃, G - NaHMDS, H - DIPEA, I - DABCO, J - DBU). The vials were then stirred in a block heated to 100 °C. The reaction was monitored with TLC (pre-coated silica plates Merck, hexane-acetone 7 : 3) and HPLC. The results are summarized in Table I.

**Table I - Cyclization with a combination of BSA and a base**

| **Entry** | **Base** | **HPLC content** | | | |
|---|---|---|---|---|---|
| | | **IIaa** | **X** | **IIIaa** | **VIII** |
| A | - | 4.2 % | 85.2 % | 3.4 % | - |
| B | BSA | - | 0.7 % | 91.7 % | 1.1 % |
| C | NaHCO₃ | 23.8 % | 67.8 % | 0.8 % | 1.0% |
| D | KHCO₃ | 17.6 % | 72.3 % | 4.2 % | - |
| E | Na₂CO₃ | 2.1 | 0.7 | 61.1 % | 13.1 % |
| F | K₂CO₃ | 0.7 | 0.4 | 54.4 % | 26.7 % |
| G | NaHMDS | - | - | 73.6 % | 12.7 % |
| H | DIPEA | 9.9 | 82.3 | 2.9 % | 0.7 |
| I | DABCO | 6.4 % | 71.4 % | 4.4 % | 1.2 % |
| J | DBU | 9.1 % | 63.8 % | 4.3 % | 3.1 % |

### Example 15

### (R)- 6-(3-Chloro-2-fluorobenzyl)-1-(1-hydroxy-3-methylbutan-2-yl)-7-methoxy-4-oxo-1,4-dihydroquinoline-3- carboxylic acid - elvitegravir (XII)

Using the procedure described in Examples 1, 8 and 12, starting from (*R*)-methyl 3-(1-hydroxy-3-methyl-butan-2-ylamino)-2-(5-bromo-2,4-dimethoxybenzoyl)-acrylate) (*R*-**IIaa**), (*R*)-6-(3-chloro-2-fluorobenzyl)-1-(1-hydroxy-3-methylbutan-2-yl)-7-methoxy-4-oxo-1,4-dihydroquinoline-3-carboxylic acid - elvitegravir (XII) was obtained, without isolation of intermediates, in the 21% yield in the form of colourless crystals with the melt point of 197-201 °C.

## Claims

1. A method of producing elvitegravir of formula **I,** 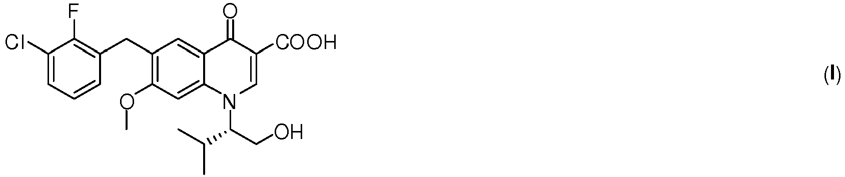 starting from dimethoxy derivatives of the general formula **II,** 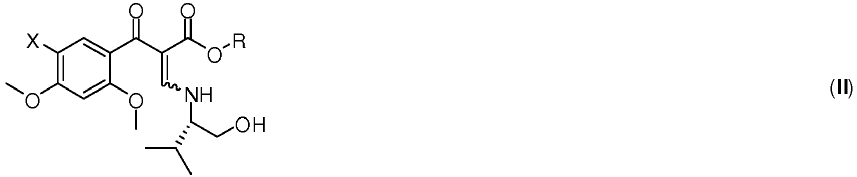 wherein X is Cl, Br, or I, and wherein R is an (un)branched C₁-C₄ alkyl, **characterized in** reacting with a suitable silylation agent, selected among *O,N*-bis-trimethylsilylacetamide, *O,N*-bis-trimethylsilyltrifluoroacetamide or *N*-methyl-trimethylsilylacetamide, in a solvent, selected among an excess of the silylation agent used or dimethyl formamide, to provide *in situ,* after processing and isolation, the corresponding silylated intermediate **III**, 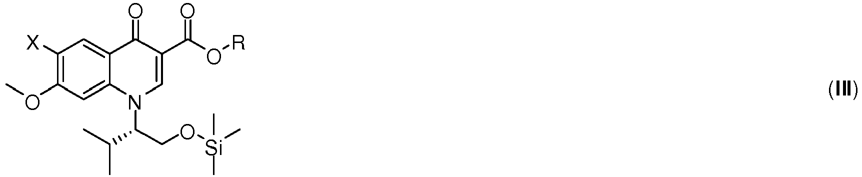 wherein X and R have the above mentioned meaning, which then provides, through a reaction with the organozinc reagent of formula **IV,** 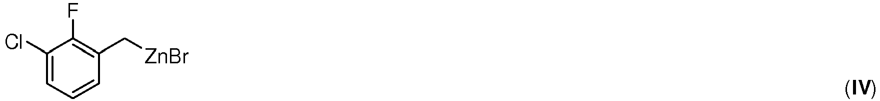 an intermediate of the general formula **V,** 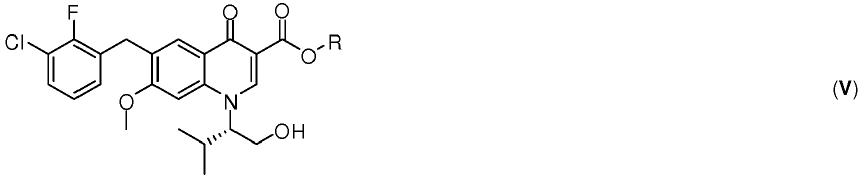 wherein R is an (un)branched C₁-C₄ alkyl, which is optionally crystallized from a crystallization solvent and further hydrolyzed to elvitegravir (**I**), which is optionally further crystallized from another crystallization solvent.

2. The method according to claim 1, **characterized in that** the reaction with the silylation reagent is carried out at a temperature in the range of 80 to 120 °C.

3. The method according to claim 2, **characterized in that** the silylation agent is *O,N*-bis-trimethylsilylacetamide.

4. The method according to claim 3, **characterized in that** *O,N*-bis-trimethylsilylacetamide is used in an amount in the range of 2.2 - 5 equivalents.

5. The method according to claim 4, **characterized in that** the processing and isolation of the silylated intermediate **III** is carried out by cooling the reaction mixture to a temperature in the range of 0-5 °C, and adding dropwise an amount of water sufficient to form a precipitate, which is filtered off and dried.

6. The method according to claim 1, **characterized in that** the crystallization solvent for crystallization of the intermediate of formula **V** is toluene or C₁-C₃ (un)branched alcohols or a mixture of toluene with said alcohols, preferably a toluene-methanol mixture.

7. The method according to claim 1, **characterized in that** the intermediate **V is** hydrolyzed to elvitegravir of formula **I,** 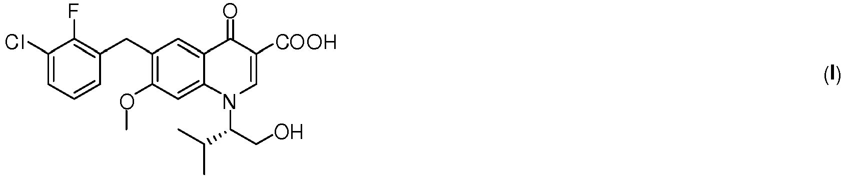 in an alkaline environment.

8. The method according to claim 7, **characterized in that** the intermediate **V** is hydrolyzed in a mixture of water : C₁-C₃ (un) substituted aliphatic alcohol in the presence of an alkali hydroxide, preferably in a water : methanol mixture in the presence of sodium hydroxide.

9. The method according to claim 8, **characterized in that** the hydrolysis is carried out at a temperature in the range from 20 °C to the boiling point of the mixed solvent.

10. The method according to claim 1, **characterized in that** the other crystallization solvent for crystallization of elvitegravir of formula **I** is a mixture of a polar organic solvent and water, preferably a mixture of acetone and water.

## Patentansprüche

1. Verfahren zur Herstellung von Elvitegravir der Formel I, 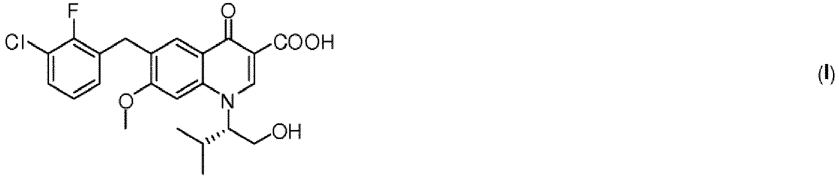 ausgehend von Dimethoxyderivaten der allgemeinen Formel II, 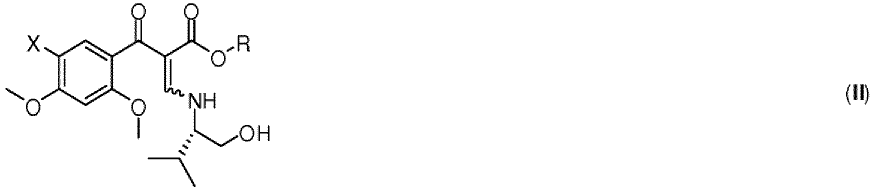 worin X Cl, Br oder I ist und worin R ein (un)verzweigtes C₁-C₄-Alkyl ist, **dadurch gekennzeichnet, dass** sie mit einem geeigneten Silylierungsmittel, ausgewählt aus *O,N*-Bis-Trimethylsilylacetamid, *O,N*-Bis-Trimethylsilyltrifluoracetamid oder N-Methyl-Trimethylsilylacetamid, in einem Lösungsmittel, ausgewählt aus einem Überschuss des eingesetzten Silylierungsmittels oder Dimethylformamid, reagieren, um, nach Aufbereitung und Isolierung, das entsprechende silylierte Zwischenprodukt III *in situ* bereitzustellen, 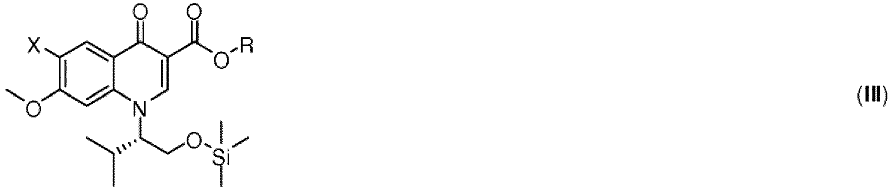 worin X und R die oben genannte Bedeutung haben, womit anschließend durch Reaktion mit dem Organozinkreagens der Formel IV 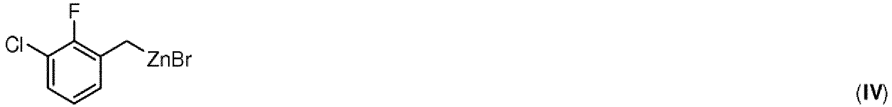 ein Zwischenprodukt der allgemeinen Formel V, 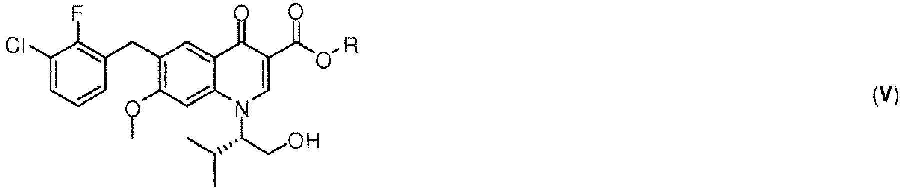 erhalten wird, worin R ein (un)verzweigtes C₁-C₄-Alkyl ist, welches gegebenenfalls aus einem Kristallisationslösungsmittel kristallisiert und weiter zu Elvitegravir (I) hydrolysiert wird, das gegebenenfalls weiter aus einem anderen Kristallisationslösungsmittel kristallisiert wird.

2. Verfahren nach Anspruch 1, **dadurch gekennzeichnet, dass** die Reaktion mit dem Silylierungsreagens bei einer Temperatur im Bereich von 80 bis 120 °C durchgeführt wird.

3. Verfahren nach Anspruch 2, **dadurch gekennzeichnet, dass** das Silylierungsmittel *O,N*-Bis-Trimethylsilylacetamid ist.

4. Verfahren nach Anspruch 3, **dadurch gekennzeichnet, dass** *O,N*-Bis-Trimethylsilylacetamid in einer Menge im Bereich von 2,2-5 Äquivalenten eingesetzt wird.

5. Verfahren nach Anspruch 4, **dadurch gekennzeichnet, dass** die Aufbereitung und Isolierung des silylierten Zwischenprodukts III durch Abkühlen des Reaktionsgemisches auf eine Temperatur im Bereich von 0-5 °C und tropfenweise Zugabe einer Wassermenge erfolgt, welche ausreicht, um einen Niederschlag zu bilden, der abfiltriert und getrocknet wird.

6. Verfahren nach Anspruch 1, **dadurch gekennzeichnet, dass** das Kristallisationslösungsmittel zur Kristallisation des Zwischenproduktes der Formel V Toluol oder (un)verzweigte C₁-C₃-Alkohole oder ein Gemisch von Toluol mit diesen Alkoholen, vorzugsweise ein Toluol-Methanol-Gemisch, ist.

7. Verfahren nach Anspruch 1, **dadurch gekennzeichnet, dass** das Zwischenprodukt V in einer alkalischen Umgebung zu Elvitegravir der Formel I 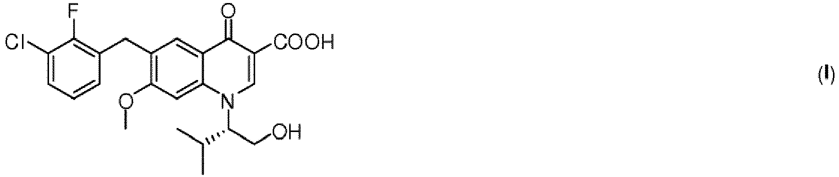 hydrolysiert wird.

8. Verfahren nach Anspruch 7, **dadurch gekennzeichnet, dass** das Zwischenprodukt V in einer Mischung von Wasser: (un) substituiertem aliphatischem C₁-C₃-Alkohol in Gegenwart eines Alkalihydroxids, vorzugsweise in einem Wasser:Methanol-Gemisch in Gegenwart von Natriumhydroxid, hydrolysiert wird.

9. Verfahren nach Anspruch 8, **dadurch gekennzeichnet, dass** die Hydrolyse bei einer Temperatur im Bereich von 20 °C bis zum Siedepunkt des Lösungsmittelgemischs erfolgt.

10. Verfahren nach Anspruch 1, **dadurch gekennzeichnet, dass** das andere Kristallisationslösungsmittel zur Kristallisation von Elvitegravir der Formel I eine Mischung aus einem polaren organischen Lösungsmittel und Wasser, vorzugsweise einer Mischung aus Aceton und Wasser, ist.

## Revendications

1. Un procédé de production d'elvitégravir de formule I, 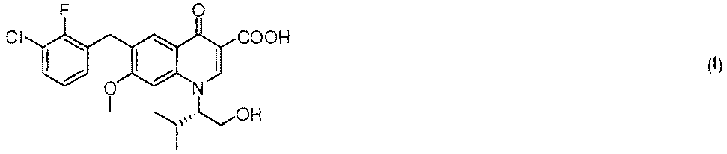 à partir de dérivés diméthoxy de formule générale II, 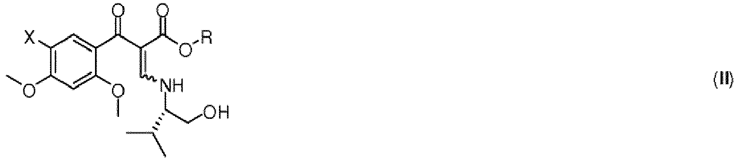
- où X est CI, Br ou I, et
- où R est un groupe alkyle en C₁-C₄ ramifié ou non ramifié,
**caractérisé par** la réaction avec un agent de silylation approprié choisi parmi O,N-bis-triméthylsilylacétamide, le O,N-bis-triméthylsilyltrifluoroacétamide ou le N-méthyl-triméthylsilylacétamide, dans un solvant choisi par an excès de l'agent de silylation utilisé ou du diméthylformamide pour fournir in situ, après traitement et isolement, le intermédiaire silylé III correspondant, 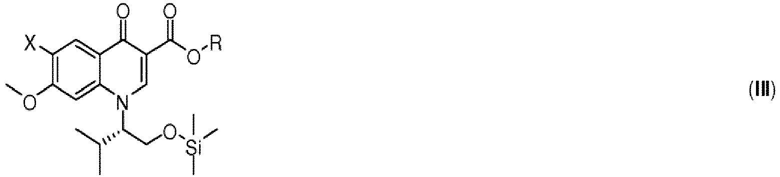
- dans lequel X et R ont la signification mentionnée ci-dessus,
qui fournit ensuite, par réaction avec le réactif organozinc de formule IV, 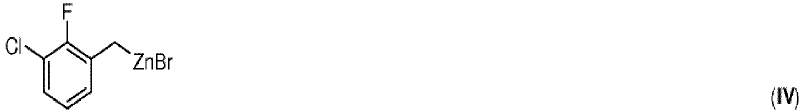 un intermédiaire de formule générale V, 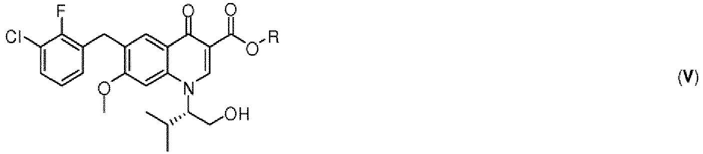 dans laquelle R est un groupe alkyle en C₁-C₄ ramifié ou non ramifié, qui est éventuellement cristallisé par action d'un solvant de cristallisation et ensuite hydrolysé en elvitégravir (I), qui est éventuellement ensuite cristallisé par action d'un autre solvant de cristallisation.

2. Le procédé selon la revendication 1, **caractérisé en ce que** la réaction avec le réactif de silylation est effectuée à une température comprise entre 80 et 120°C.

3. Le procédé selon la revendication 2, **caractérisé en ce que** l'agent de silylation est le O,N-bis-triméthylsilylacétamide.

4. Le procédé selon la revendication 3, **caractérisé en ce que** le O,N-bis-triméthylsilylacétamide est utilisé en une proportion comprise entre 2,2 et 5 équivalents.

5. Le procédé selon la revendication 4, **caractérisé en ce que** le traitement et l'isolement de l'intermédiaire silylé III sont effectués par refroidissement du mélange réactionnel à une température comprise entre 0 et 5°C et addition goutte à goutte d'une quantité d'eau suffisante pour former un précipité, qui est séparé par filtration et séché.

6. Le procédé selon la revendication 1, **caractérisé en ce que** le solvant de cristallisation pour la cristallisation de l'intermédiaire de formule V est du toluène ou des alcools en C₁-C₃, ramifiés ou non ramifiés ou un mélange de toluène avec lesdits alcools, de préférence un mélange toluène-méthanol.

7. Le procédé selon la revendication 1, **caractérisé en ce que** l'intermédiaire V est hydrolysé en elvitégravir de formule I, dans un environnement alcalin.

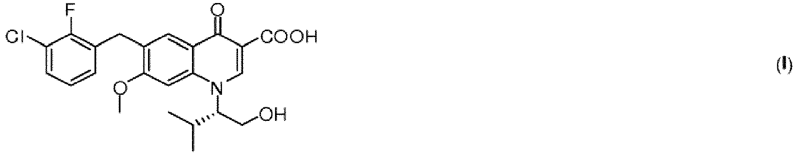

8. Le procédé selon la revendication 7, **caractérisé en ce que** l'intermédiaire V est hydrolysé dans un mélange d'eau/alcool aliphatique en C₁-C₃ substitué ou non substitué en présence d'un hydroxyde alcalin, de préférence dans un mélange eau/méthanol en présence d'hydroxyde de sodium.

9. Le procédé selon la revendication 8, **caractérisé en ce que** l'hydrolyse est effectuée à une température comprise entre 20°C et le point d'ébullition du solvant mélangé.

10. Le procédé selon la revendication 1, **caractérisé en ce que** l'autre solvant de cristallisation pour la cristallisation de l'elvitégravir de formule I est un mélange d'un solvant organique polaire et d'eau, de préférence un mélange d'acétone et d'eau.
